# EUROPEAN PATENT SPECIFICATION

(11) **EP 1 402 942 B1**
(45) Date of publication and mention of the grant of the patent: **24.02.2010**
(21) Application number: 01930163.9
(22) Date of filing: 15.05.2001
(51) Int. Cl.: C07C 51/265, B01J 8/00, B01J 19/00

(54) **PROCESS FOR PRODUCING A COMPOUND**
VERFAHREN ZUR HERSTELLUNG EINER VERBINDUNG
PROCEDE DE PRODUCTION D'UN COMPOSE

(43) Date of publication of application: 31.03.2004
(73) Proprietor: Mitsubishi Chemical Corporation, Minato-ku Tokyo 108-0014 (JP); TOMOE ENGINEERING CO. LTD., Chuo-ku, Tokyo 103-0027 (JP)
(72) Inventor: NUMATA, Motoki Mitsubishi Chemical Corporation, Kitakyushu-shi Fukuoka 806-0004 (JP); IWASAKI, Toshiya Mitsubishi Chemical Corporation, Kitakyushu-shi Fukuoka 806-0004 (JP); FUKUDA, Katsunori Mitsubishi Chemical Corporation, Kitakyushu-shi Fukuoka 806-0004 (JP); ISOGAI, Takayuki Mitsubishi Chemical Corporation, Kitakyushu-shi Fukuoka 806-0004 (JP); ANDO, Katsuya, Shinagawa-ku Tokyo 141-0032 (JP)
(74) Representative: Vossius & Partner
(86) International application number: PCT/JP2001/004047
(87) International publication number: WO 2002/092214

(56) References cited:
- WO-A-97/24311
- WO-A1-00/71226
- US-A- 5 095 146
- US-A- 5 175 355
- US-A- 5 741 927
- US-E- R E36 008

## Description

The present invention relates to a process of producing a compound obtained by reaction under pressure and heat, including a step of removing inclusions such as a reaction medium and/or a washing liquid from the compound utilizing internal energy.

The case where a desired solid particulate product is obtained as a slurry that is a mixture with a reaction mother liquor is found here and there in the chemical process. In general, the solid particulate product is obtained when the slurry passes through unit operations of separation and drying.

Hitherto, a number of attempts for improving the foregoing unit operations to enhance the process have been made. For example, with respect to the drying operation, JP-A-52-59177 describes an example of drying by external heating with a hot gas or hot air using a compressed air transfer type drying machine. Also, JP-B-58-11418 and JP-A-55-164650 describe an example of obtaining a solid and a gas by evaporating a slurry liquid within a heating pipe. However, since these technologies are to dry a cake by newly giving a heat on the assumption that the independent drying operation is carried out, it was necessary to use energy corresponding to drying.

On the other hand, it is general to carry out crystallization by decreasing the temperature while keeping the slurry state as a pre-treatment of solid-liquid separation. For example, British Patent No. 1,152,575 describes an example in which a solvent is evaporated to cause cooling, thereby precipitating terephthalic acid. However, the evaporation itself merely increases the slurry concentration a little, but there is not observed any effect other than a decrease of the temperature against the process.

Also, JP-A-11-33532 describes an example in which terephthalic acid is slurried with a washing liquid and flashed. Since it is difficult to discharge a powder in a pressurized state, a method of re-slurrying the powder and then discharging it is generally known. However, a negative part where energy has been lost was not watched. Accordingly, it is hard to say that the process of decreasing the slurry temperature to get the energy scattered and lost and again heating by drying, as described in the foregoing two examples, effectively utilizes the energy.

US-A-5,175,355 discloses a process for preparation of purified terephthalic acid containing 200 ppmw or less of p-toluic acid. A filter cake of purified terephthalic acid is prepared by filtering, under a differential pressure of about or greater than 0.5 psi over the system pressure and a temperature within the range of from about 100° C. to about 205° C., an aqueous slurry of purified terephthalic acid containing a solution of p-toluic acid. The aqueous solution of p-toluic acid remaining in the filter cake of purified terephthalic acid is displaced from the filter cake by water under a pressure gradient over the system pressure at a temperature within the range of from about 100° C. to about 205° C. Pressure flash evaporation of water remaining in the filter cake occurs upon release of the system pressure to lower pressure with consequent lower temperature. The crystalline terephthalic acid product containing 200 ppmw or less p-toluic acid can be dried under atmospheric pressure. Purified terephthalic acid is useful for the manufacture of polyesters from which clothing and related goods are made.

WO-A-00/71226 describes an assembly for continuous pressure filtration of a slurry, the assembly comprising a pressurized filtration unit comprising a vessel maintained at a first pressure, filter media in said vessel for separating said slurry into a liquid phase material and a solid phase material, a liquid outlet for discharge of said liquid phase material, and a solids outlet for discharge of said solid phase material from said filtrationunit; a depressurizing chamber maintained at a second and lower pressure than said first pressure, said chamber having a solids inlet receiving said solid phase material from said filtration unit and containing the solid phase material as it is exposed to said second and lower pressure; and a material transport between said solids outlet of said filtration unit and said solids inlet of said chamber for maintaining the pressure difference between said filtration unit and said chamber and for continuously moving said solid phase material from said solids outlet of said filtration unit into said chamber; whereby said solid phase material is depressurized from about said first pressure to said second andlower pressure.

Also, JP-A-1-299618, U.S. Patent No. 5,698,734, and WO 91/09661 illustrate the cake separation in a pressurized state. However, it is not mentioned at all that to keep heat energy of the slurry before separation is effective for cake drying.

Also, JP-T-60-506461 describes an example in which a slurry of terephthalic acid and water are separated under pressure, and the moisture remaining in the cake is subjected to flash evaporation. However, since an evaporation latent heat of water is high, it is possible to evaporate only a part of the cake-attached liquid. In order to take out the compound, it is necessary to pass through the usual drying step.

An object of the invention is to reduce the use of energy in the drying step by utilizing internal energy that the slurry after reaction has, and especially preferably to greatly reduce energy to be used by drying the cake only by internal energy that the slurry has and to construct a simple process by integrating the separation/drying step.

In order to solve the foregoing problems, the present inventors made extensive and intensive investigations. As a result, it has been found that when certain production steps are employed, internal energy that a reaction product obtained by reaction under pressure and heating has an ability to evaporate the greater part of a cake-attached liquid, leading to accomplishment of the invention.

The present invention relates to a process of producing a compound, including:
(A) a reaction step, wherein a raw material and a reaction medium are fed into a reactor (1) and wherein in the reactor (1) a reaction is carried out under a pressure higher than atmospheric pressure and at a temperature of the boiling point at atmospheric pressure of the reaction medium or higher, to form a compound,
   wherein the reaction medium is one that becomes a liquid phase system or a gas-liquid two-phase system during the reaction step, and one that does not chemically change the reaction substrate or the desired compound after the reaction;
(B) a separation step, wherein in a separation device (3) a slurry containing the compound and the reaction medium is subjected to solid-liquid separation under a pressure higher than atmospheric pressure and at a temperature of the boiling point at atmospheric pressure of the reaction medium or higher, to obtain a cake having a weight ratio of a cake-attached liquid of not more than 50 %, based on the solids content, wherein the cake-attached liquid is the sum of the reaction medium and a washing liquid in the optional case of washing the cake;
(C) a drying step, wherein the resulting cake is moved into a compound recovery zone (6) having a pressure lower than the pressure in the separation device (3) and a temperature lower than the temperature in the separation device (3), thereby evaporating the cake-attached liquid by internal energy released during the movement,
   wherein in the drying step (C), the weight ratio of the cake-attached liquid is not more than 10 % based on the solids content; and
(D) optionally an intermediate processing step between the reaction step (A) and the separation step (B);
   wherein the steps from the reactor (1) until the separation device (3) optionally through an intermediate processing tank (2) meet the requirements that the pressure be kept higher than atmospheric pressure and the temperature be kept at the boiling point at atmospheric pressure of the reaction medium or higher.

Specifically, the gist of the invention resides in a process of producing a compound including at least the following steps:
(A) a reaction step of undergoing reaction in a reactor under a pressure higher than atmospheric pressure and at a boiling point at atmospheric pressure of a reaction medium or higher, to form a compound;
(B) a separation step of separating a fixed amount or more of the reaction medium from a slurry containing the compound and the reaction medium under a pressure higher than atmospheric pressure and at a temperature of a boiling point at atmospheric pressure of the reaction medium or higher in a separation device, to obtain a cake having a weight ratio of a cake-attached liquid of not more than 50 % based on the solids content and
(C) a drying step of moving the resulting cake into a compound recovery zone having a pressure lower than the pressure in the separation device and a temperature lower than the temperature in the separation device, thereby evaporating the cake-attached liquid by internal energy released by the movement.

Fig. 1 is a schematic view of a process example for carrying out the invention.
Fig. 2 is a schematic view of one example of a preferred flash tank for carrying out the invention.
Fig. 3 is a schematic view of a flash tank used in the Examples.

Incidentally, reference numerals and signs in the drawings are as follows. 1 is a reactor; 2 is an intermediate processing tank; 3 is a separation device; 4 is a chamber; 5 is a discharge valve; 6 is a powder tank (compound recovery zone); 7 is a chute; 8 is an approximately conical valve body; 9 is an upper valve; 11 is an introduction line of raw material, etc.; 12 is reaction product transfer line; 13 is a reaction product transfer line; 14 is an evaporation gas discharge line; 15 is a compound recovery line; 16 is an intermediate chamber; 21 is a detector; 22 is a seal; 23 is a seal; 24 is a cylinder; 31 is an upper valve of heater; 32 is a heater; 33 is a bottom valve of heater; 34 is a receiver (reaction product recovery zone); 35 is an oil bath; 36 is a valve between receiver and trap; 37 is trap; and 38 is a jacket, respectively.

The invention will be hereunder described in detail.

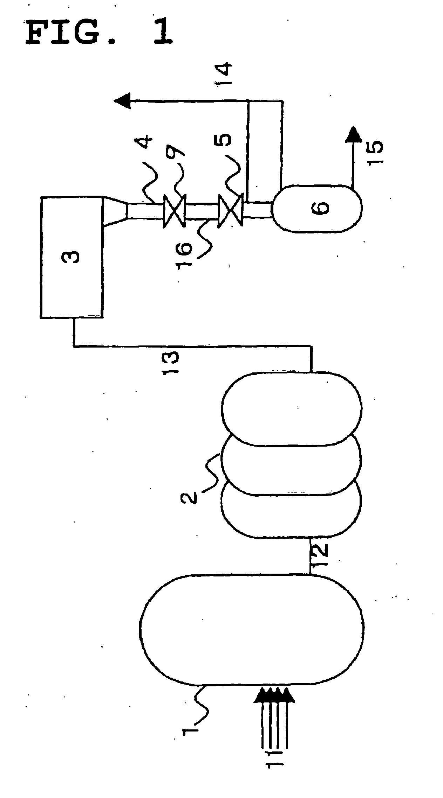

Fig. 1 is a conceptual view for explaining a specific method of the invention.

A raw material and a reaction medium are fed into a reactor 1 through a line 11. Reaction is carried out in the reactor under a fixed condition. A reaction product after the reaction may be comprised of a liquid one-phase system, a gas-liquid two-phase system, a solid-liquid two-phase system, or a gas-liquid-solid three-phase system. Especially, one comprised of a solid-liquid two-phase system or a gas-liquid-solid three-phase system is called a slurry. In the production process of the invention, for the sake of undergoing solid-liquid separation in the separation step, it is necessary that the reaction product be kept as the slurry until it has been transferred into the separation device.

The desired compound is obtained as a solid, and preferably as a crystal, and the slurry containing at least the solid compound and the reaction medium is obtained. Incidentally, a part of the desired compound may be dissolved in the reaction medium.

The reaction product is transferred into an intermediate processing tank 2 through a line 12 and subjected to crystallization, dissolution, or other intermediate processing as the need arises. The reaction product having been subjected to intermediate processing is transferred into a separation device 3 through a line 13. In the case where the reaction product is not subjected to intermediate processing, the reaction product is directly transferred into the separation device from the reactor 1.

In the separation device 3, the solid and the liquid in the slurry are separated to prepare a cake having a weight ratio of a cake-attached liquid of not more than 50 %, preferably not more than 30 %, more preferably not more than 20 %, and especially preferably not more than 15 % based on the solids content (in the present description, a weight ratio (W1/W2) of the cake-attached liquid (W1) to the solids content (W2) being hereinafter referred to as "liquid content or content of the cake-attached liquid"). The cake is washed with a washing liquid within the separation device as the need arises.

The cake prepared in the separation device 3 is transferred into a powder tank 6 (reaction product recovery zone) through a chamber 4 and a valve 5, whereby the desired compound is obtained as a solid.

Next, in the production process of the invention, each step of the reaction step (A), the separation step (B), the drying step (C), and the intermediate processing step (D) to be added as the need arises will be described.

### (A) Reaction step:

In the production process of the invention, the reaction for forming a compound is carried out under a pressure higher than atmospheric pressure and at a temperature of a boiling point at atmospheric pressure of a reaction medium or higher. A raw material is fed into the reactor 1 through the line 11. The line 11 may be single or plural according to the feed condition.

The raw material may be any of a gas, a liquid, a solid, or a slurry and may be of a single phase system or a mixed phase system. Further, the composition may be of a pure substance or a mixture, but usually, a reaction medium and optionally a catalyst and additives are added to a reaction substrate. Such compositions are arbitrarily determined depending upon the kind of reaction. The temperature is also arbitrarily determined but usually is within the range where the reaction substrate and the reaction medium do not decompose. Moreover, the phase state of the raw material may be intentionally changed according to the temperature. For example, there is enumerated an example in which the slurry is converted into a completely dissolved phase by increasing the temperature, or the liquid phase is converted into a liquid-gas mixed phase.

As the reaction mode to be used in the invention, any of oxidation reaction, reduction reaction, displacement reaction, addition reaction, elimination reaction, or the like may be employed. Also, the reaction may be exothermic reaction or endothermic reaction, but exothermic reaction is preferable from the viewpoint of effectively utilizing internal energy. Moreover, the compound formed in the reaction vessel may be liquid but is preferably obtained as a solid, and more preferably as a crystal.

Also, the reaction may be carried out in a batchwise manner or a continuous manner.

The reaction medium that is used in the invention is one that becomes a liquid phase system or a gas-liquid two-phase system during the reaction step, and one that does not chemically change the reaction substrate or the desired compound after the reaction is used. Also, an evaporation latent heat of the reaction medium at atmospheric pressure (in the invention, when the evaporation latent heat is defined, one at atmospheric pressure is referred to hereinafter) is preferably not more than 300 kcal/kg, more preferably not more than 200 kcal/kg, and especially preferably not more than 150 kcal/kg. Also, the lower limit of the evaporation latent heat is not defined but is usually 50 kcal/kg or more, and preferably 70 kcal/kg or more.

Also, the boiling point at atmospheric pressure of the reaction medium is preferably from 40 °C to 200 °C, more preferably from 50 °C to 180 °C, and especially preferably from 60 °C to 150 °C. In the case where the boiling point at atmospheric pressure of the reaction medium is remarkably lower than the foregoing range, the handling and recovery of the reaction medium in each of the steps tend to become difficult. On the other hand, in the case where it is remarkably high, the residual amount of the reaction medium during moving the desired compound into the compound recovery zone in the separation step tends to increase.

In the reactor 1, when the raw material is fed, 30 % or more, preferably 50 % or more, and more preferably 70 % or more, for example, 80 % or more of the subjective reaction substrate is chemically changed. Examples of the reaction mixture include various systems such as a liquid one-phase system, a gas-liquid two-phase system, and a gas-liquid-solid three-phase system.

The reaction condition is determined while taking into account various factors such as reaction rate, side reactions, and solubility in the reaction medium. The reaction is usually carried out at a temperature in the range of from 50 °C to 350 °C, preferably from 100 °C to 300 °C, more preferably from 130 °C to 250 °C, and especially preferably from 150 °C to 230 °C and at a temperature of the boiling point at atmospheric pressure of the reaction medium or higher. Also, the reaction is carried out under a pressure in the range of more than atmospheric pressure but not higher than 20 MPa, preferably from 0.2 MPa to 10 MPa, more preferably from 0.5 MPa to 5 MPa, and especially preferably from 1 MPa to 3 MPa. In the case where the reaction temperature and pressure are remarkably lower than the foregoing ranges, the internal energy that the cake obtained during the production steps of the invention is small, and the evaporation of the cake-attached liquid during the movement of the cake into the compound recovery zone is not sufficient. Also, in the case where the reaction temperature and pressure are remarkably higher than the foregoing ranges, side reactions are liable to take place, or the compound is liable to decompose, whereby the yield tends to become low.

In the production process of the invention, a catalyst may be used during the reaction step. The catalyst may be a heterogeneous catalyst or a homogeneous catalyst. Temperature control of the reactor is carried out by heating or heat removal. This is determined by the temperature of feeding liquid, the kind of reaction such as endothermic reaction and exothermic reaction, and the like. The heating is carried out using a jacket or a coil, and the heat removal can be effected by further evaporation operation.

In the reaction step, the case where the reaction condition changes on the way (including the case of a plurality of reactors) may be included. In this case, the definition as the reaction condition of the reaction step of the invention (under a pressure higher than atmospheric pressure and at a temperature of the boiling point at atmospheric pressure of the reaction medium or higher) refers to the reaction condition most closely to the separation step from the time standpoint. Accordingly, the production process of the invention is distinguished from a production process in which reaction is carried out under a pressure not higher than atmospheric pressure or at a temperature not higher than a boiling point at atmospheric pressure of a reaction medium, and after completion of the reaction, when the reaction mixture reaches a separation device under pressure and/or heating, a pressure higher than atmospheric pressure and a temperature of the boiling point at atmospheric pressure of the reaction medium or higher are brought.

Incidentally, the steps of from the reactor until the separation device through the intermediate processing tank to be used as the need arises may include an additional unit operation such as dilution and heating so far as they meet the requirements that the pressure be kept at a pressure higher than atmospheric pressure and that the temperature be kept at the boiling point at atmospheric pressure of the reaction medium or higher.

### (D) Intermediate processing step:

The intermediate processing step is not essential, and a plural number of intermediate processing tanks may be provided. Examples of the intermediate processing step include cooling, heating, pressure rising, pressure reduction, concentration, dilution, precipitation, addition, and the like. Typically, crystallization or dissolution is carried out. For example, in the case where it is intended to increase the percent recovery of the compound, crystallization is carried out, and when it is intended to increase the purity of the compound, dissolution is carried out. Such intermediate processing steps are properly chosen depending on the kind of the desired compound, the product specification, and the like.

Incidentally, in the case where the reaction product obtained by the reaction step is in a solid-free state such as a supersaturated state, a liquid one-phase system, and a gas-liquid two-phase system, it is essential to solidify the desired compound prior to the separation step, and preferably, crystallization is carried out in the intermediate processing step.

Also, the intermediate processing step does not always require the use of a vessel such as an intermediate processing tank but may be carried out in the line of the movement from the reaction step into the separation step.

In the case where crystallization is carried out as a preferred example of the operation in the intermediate processing step, the number of stages of crystallization is preferably not more than four stages, more preferably not more than two stages, and especially preferably one stage. In any places of the line 12, the intermediate processing tank 2, and the line 13, the reaction product keeps a temperature of the boiling point at atmospheric pressure of the reaction medium or higher and a pressure higher than atmospheric pressure. The pressure range preferably exceeds atmospheric pressure but is not higher than 20 MPa, more preferably from 0.2 MPa to 10 MPa, and especially preferably from 0.3 MPa to 5 MPa. The temperature range is usually from 50 °C to 350 °C, preferably from 100 °C to 300 °C, and more preferably from 130 °C to 250 °C.

A difference (TD - Bp1) between the boiling point (Bp1) at atmospheric pressure of the reaction medium and the temperature (TD) of the reaction product in the intermediate processing step 2 is preferably in the range of from 5 °C to 200 °C, more preferably from 10 °C to 150 °C, and especially preferably from 15 °C to 100 °C. By keeping the foregoing temperature difference, it is possible to make at least a part of heat energy that the reaction product discharged from the line 12 has remain in the reaction product of the line 13. Incidentally, as described previously, the reaction product may be comprised of any of a liquid one-phase system, a gas-liquid two-phase system, a solid-liquid two-phase system, or a gas-liquid-solid three-phase system. Especially, one comprised of a solid-liquid two-phase system or a gas-liquid-solid three-phase system is called a slurry. In the production step of the invention, the reaction product usually becomes a slurry at the time when it moves in the line 13.

### (B) Separation step:

The reaction product in the slurry state is moved into the separation device 3 from the intermediate processing tank 2 through the line 13, or from the reactor 1 through another line (not shown).

The separation device 3 is not particularly limited in terms of mechanism, but ones that are generally known as described in JP-T-7-507291, such as centrifugal separators, horizontal belt filters, and rotary vacuum filters, and filter cells described in JP-T-6-502653 can be used. Especially, in the case where a centrifugal separator is used, it can be selected from screen bowl decanters and solid bowl decanters.

In order that the heat energy that the slurry entering the separation device 3 through the line 13 has is not completely scattered and lost, the pressure and temperature of the separation device 3 are adjusted. When the pressure of the separation device 3 is low, the slurry causes flash so that the temperature is lowered. Accordingly, the pressure of the separation device 3 is atmospheric pressure or higher and if desired, may be higher than the pressure of the reactor 1 or the intermediate processing tank 2.

The slurry moved into the separation device is subjected to solid-liquid separation within the separation device, and the content of the reaction medium in the slurry based on the solids content is reduced to not more than 50 %, and preferably not more than 30 %, to obtain a cake. In the production process of the invention, the desired compound as a solid constitutes the major component of the cake, in which the reaction medium or a washing liquid in the case of washing the cake as described later (in the present description, the sum of the reaction medium and the washing liquid present in the cake is called as a cake-attached liquid) is contained in a content in the range of not more than 50 %, and preferably not more than 30 % based on the solids content.

It is preferable that the temperature (TB2) of the cake obtained in the separation device 3 immediately before discharge is higher than the boiling (Bp2) at atmospheric pressure of the cake-attached liquid immediately before discharging from the separation device 3. For example, in the case where a slurry is transferred into the separation device 3 having a high pressure through the line 13, it is possible to realize the transfer by rising the pressure by a pump or the like before entering the separation device 3. The pressure range of the separation device 3 is preferably from 0.11 MPa to 22 MPa, more preferably from 0.21 MPa to 12 MPa, and especially preferably from 0.31 MPa to 7 MPa. The temperature range of the cake immediately before the discharge is from 50 °C to 350 °C, preferably from 100 °C to 300 °C, and more preferably from 130 °C to 250 °C. A difference (TB2 - Bp2) between the boiling point at atmospheric pressure of the cake-attached liquid immediately before discharging from the separation device 3 and the temperature of the cake to be discharged from the separation device 3 is preferably in the range of from 5 °C to 200 °C, more preferably from 10 °C to 150 °C, and especially preferably from 15 °C to 100 °C.

Also, it is possible to add a washing function to the separation device 3. The kind of the washing liquid is not particularly limited but may be a component quite different from the reaction medium or inversely, may be exactly the same as the reaction medium. Also, the presence or absence of slurrying in washing is not particularly limited. Here, in order that the heat energy of the separated cake is not scattered and lost, the amount and temperature of the washing liquid are controlled. In the case of using a washing liquid having a low temperature, the amount of the washing liquid is made low so as to suppress the influence. If the heat removal from the cake can be inhibited by rising the temperature of the washing liquid, it is possible to increase the amount of the washing liquid. Accordingly, a specific temperature of the washing liquid is in the range of higher than the boiling point at atmospheric pressure of the washing liquid but not higher than (TB1 + 100 °C) (wherein TB1 stands for the temperature of an unwashed cake, which is in general substantially equal to the temperature in the separation device before washing), preferably from TB1 to (TB1 + 80 °C), and more preferably from (TB1 + 5 °C) to (TB1 + 60 °C).

Also, a specific amount of the washing liquid is preferably from 0.03 to 5.0, more preferably from 0.05 to 4.0, and especially preferably from 0.1 to 3.0 in terms of a weight ratio based on the solids content in the cake. Incidentally, for the sake of preventing bumping of the washing liquid, the pressure within the separation device into which the washing liquid is introduced is a vapor pressure of the washing liquid or higher. The pressure within the separation device is higher than the vapor pressure of the slurry to be fed into the separation device by the range of preferably from 0.01 to 2.0 MPa, more preferably from 0.01 to 1.0 MPa, and especially preferably from 0.02 to 0.5 MPa.

The kind of the washing liquid is not particularly limited, except for the matter that the liquid be present under the pressure and temperature condition in the separation device. Also, the composition of the washing liquid may be quite different from the reaction medium, and there is no limitation at all in the evaporation latent heat of the reaction medium.

The evaporation latent heat of the washing liquid is preferably not more than 300 kcal/kg, more preferably not more than 200 kcal/kg, and especially preferably not more than 150 kcal/kg. Also, the lower limit of the evaporation latent heat is not particularly defined but is preferably low as far as possible if it can be used as the washing liquid. The lower limit of the evaporation latent heat is usually 50 kcal/kg or more, and preferably 70 kcal/kg or more.

Also, the boiling point at atmospheric pressure of the washing liquid is preferably from 40 °C to 200 °C, more preferably from 50 °C to 180 °C, and especially preferably from 60 °C to 150 °C. In the case where the boiling point at atmospheric pressure of the washing liquid is remarkably lower than the foregoing range, handling of the washing liquid tends to become difficult. In the case where it is remarkably high, the residual amount of the washing liquid during moving the desired compound into the compound recovery zone in the separation step tends to increase.

Incidentally, the liquid content of the cake to be washed as the need arises (the weight ratio of the attached liquid in the cake based on the solids content) is not more than 50 %, preferably not more than 30 %, more preferably not more than 20 %, and especially preferably not more than 15 %.

### (C) Drying step:

The form of the drying device to be used in the invention is not particularly limited so far as the respective operations of the drying step as described later can be carried out, but pressure drying devices provided with a discharge valve (hereinafter sometimes simply referred to as "valve") are usually used.

The separated cake is kept in the chamber 4 and then discharged into the powder tank 6 (compound recovery zone) through the valve 5 while controlling the powder surface as the need arises. The pressure in the chamber 4 is substantially equal to that in the separation device 3. Also, the pressure in the powder tank 6 is lower than that in the separation device 3 and is preferably atmospheric pressure. By releasing the cake under pressure within the chamber 4 into atmospheric pressure through the valve 5, the boiling point of the cake-attached liquid decreases, whereby the cake-attached liquid is evaporated by a sensible heat due to the boiling point difference. Accordingly, in the invention, it is important to make internal energy capable of being utilized as a sensible heat retain consistently by the reaction. For this reason, with respect to the reaction product, the cake, the desired compound, and the like, the temperatures and pressures in the reaction step (A), the intermediate processing step (D), the separation step (B), and the drying step (C) until passing through the valve 5 are each kept at a temperature of the boiling point at atmospheric pressure of each of the liquids present therein or higher and at a pressure higher than atmospheric pressure, respectively.

With respect to the method of discharging the cake, there are no particular limitations about the amount of the cake to be stored in the chamber 4 and the discharge frequency. The cake may be always resident within the chamber 4 or may become empty intermittently. Incidentally, though it is possible to always open the valve 5 while maintaining a high pressure state of the chamber 4 against the powder tank 6, it is preferable to intermittently open and close the valve 5 because gas sealing properties are essential. The measurement of the amount of the cake within the chamber 4 is not particularly limited but may be carried out directly or indirectly. In general, for the sake of detecting the position of the cake level, electric contact type detectors or distance measuring equipment utilizing light or sound wave are used.

A single valve or a plural number of valves may be used as the discharge valve into the compound recovery zone from the separation device 3. In the case of a plural number of valves, it is possible to add other valve before the valve 5 most closely to the compound recovery zone. Examples of the valve 5 or 9 include a ball valve, a butterfly valve, a rotary valve, a flap damper, a slide damper, a spindle type valve, and the like. In the case of discharge using two valves as illustrated by the valves 5 and 9, for example, by alternately opening and closing the valves 5 and 9, it is possible to intermittently store the cake in an intermediate chamber 16 and discharge it through the valve 5. Also, if desired, the cake may be discharged through the valve 5 by sealing the intermediate chamber 16 and properly carrying out heating, cooling, pressure rising, pressure reduction, or gas displacement. In an embodiment of continuously discharging the cake, the opening timing of the valve 9 and the opening timing of valve 5 are controlled such that the both do not overlap each other, and the opening time is preferably from 0.5 seconds to 20 seconds, more preferably from 1 second to 15 seconds, and especially preferably from 2 seconds to 10 seconds. Specifically, there are enumerated use of a heat exchanger, gas injection, gas purge, and the like. Also, by further adding valves to increase the number of intermediate chambers to be connected in series and control the timing of opening and closing the valves, it is possible to reduce a pressure difference before and after the respective valves.

These valves are characterized by the shape of the sealing portion, examples of which include face contact and line contact. The shape of the line contact includes a rectangular shape and a circular shape. As application examples of the invention, in the case where a plural number of valves are used so as to provide intermediate chambers, the shape of the sealing portion is not particularly limited. However, in the case of discharging the compound into the compound recovery zone from the separation device using a single valve, it is preferable to use a valve in which the sealing portion is of a line contact type and is circular. Specifically, a spindle type valve in which a valve body thereof is of an approximately conical form is used. In the case where the sealing portion is of a line contact type, a sliding portion is little, and an effect of inhibiting surrounding of a solid is brought. In addition, in the case of a spindle type valve having a circular sealing portion and having an approximately conical valve body, it is possible to undergo high-speed driving.

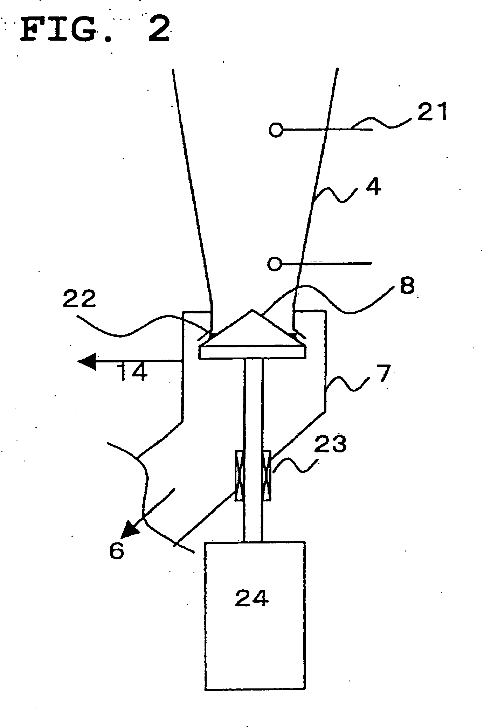

In the case of a single valve, when the opening time is long in the drying step, the pressures in the separation step and the preceding step are leaked. Accordingly, it is preferable to properly control the opening time, and for example, there is enumerated an operation of repeating opening and closure. The opening time is preferably from 0.01 to 1 second, more preferably from 0.02 to 0.5 seconds, especially preferably from 0.04 to 0.2 seconds, and most preferably from 0.05 to 0.15 seconds. In addition, a pressure drying device provided with a valve of this type is shown in Fig. 2.

The cake discharged from the separation device is transferred into the chamber 4. According to the illustration of Fig. 2, the chamber 4 is partitioned from a chute 7 by a seal 22 provided on the approximately conical valve body. After storing a proper amount of the cake by a detector 21, a cylinder 24 connected to an oil unit is driven to lower the valve 5, and an open portion is formed in the seal 22 to discharge the cake. The discharged cake is transferred into the powder tank through the chute. Also, the chute 7 is blocked from the air by a seal 23. The inner surfaces of the chamber 4 and the chute 7 are subjected to processing of preventing adhesion as the need arises. For the seal 22, the quality and shape capable of withstand high temperature, high stress and friction are chosen.

Also, in addition to the method of completely blocking the chamber and the powder tank using a valve, there is enumerated a method of using a screw conveyor in place of the discharge valve to undergo sealing with the cake within the screw conveyor. In this case, the cake is transferred into powder tank from the chamber by the screw conveyor.

A part or the whole of the cake-attached liquid is removed from the cake transferred into the compound recovery zone by evaporation due to a temperature difference. For the sake of preventing re-contamination with the evaporated cake-attached liquid, it is preferable to introduce a dry gas into the compound recovery zone or the intermediate chamber 16. Any dry gases that are inert against the compound and the attached liquid and are not saturated in the composition of the attached liquid can be used. For example, nitrogen gas, and noble gases such as argon and neon can be used.

During the movement of the cake in the chamber 4 into the powder tank 6, the attached liquid is evaporated by the released internal energy, whereby the liquid content of the cake is lowered preferably by 3 % or more, more preferably 6 % or more, and especially preferably 9 % or more. Here, what the liquid content of the cake is lowered by 3 % means that the liquid content of the cake decreases, for example, from 15 % to 12 %. Also, it is possible to decrease the liquid content of the cake to not more than 10 %, preferably not more than 5 %, and more preferably not more than 2 %. The cake is preferably stored as a powder having a low liquid content in the powder tank.

The cake-attached liquid is composed of the reaction medium, washing liquid, reaction by-products, reaction substrate, other additives, etc., or a mixture thereof, but is mainly composed of the reaction medium or washing liquid or a mixture thereof. Those cake-attached liquids having an evaporation latent heat at atmospheric pressure of not more than 300 kcal/kg, especially preferably not more than 250 kcal/kg, and further preferably not more than 200 kcal/kg, for example, not more than 150 kcal/kg are effective in the invention. In the ease where the cake-attached liquid is a mixture, this evaporation latent heat is determined as an average value of the mixture. The attached liquid is evaporated to become a gas, and the evaporated gas is discharged through a line 14 and then recovered as the need arises.

The desired compound discharged into the powder tank 6 is recovered through a line 15. When the liquid content exceeds the tolerable range as a product, it is necessary to pass it through a drying machine. In such case, it is preferable to introduce a dry gas into the intermediate chamber 16 or powder tank 6 to dry the compound, without carrying out a heat drying operation.

A difference between the temperature of the cake within the separation device and the temperature of the cake discharged into the compound recovery zone is preferably from 5 °C to 250 °C, more preferably from 10 °C to 200 °C, and especially preferably from 20 °C to 170 °C. In the case where the temperature difference is remarkably smaller than the foregoing range, the drying is insufficient, whereas in the case where it is remarkably larger than the foregoing range, the temperature of the cake within the separation device becomes too high, resulting in possible occurrence of decomposition of the compound. Also, a difference between the pressure within the separation device and the pressure in the compound recovery zone is preferably from 0.01 MPa to 22 MPa, more preferably from 0.11 MPa to 12 MPa, and especially preferably from 0.21 MPa to 7 MPa. In the case where the pressure difference is remarkably smaller than the foregoing range, the discharge of the cake becomes difficult, whereas in the case where it is remarkably higher than the foregoing range, the discharge becomes abrupt so that it is hardly controlled.

The cake discharged from the separation device preferably has a volume mean particle size of 40 µm or more, more preferably 50 µm or more, and especially preferably 60 µm or more. In the case where the volume mean particle size is remarkably smaller than the foregoing range, liquid removal becomes worse. The upper limit is not particularly defined but is usually not more than 30 µm.

Incidentally, the relative relationship between the temperature and the pressure in the foregoing reaction step (A), intermediate processing step (D) and separation step (B) is not limited, except for the matter that each of the steps keeps a temperature higher than the boiling point at atmospheric pressure of each liquid or higher and that each of the steps keeps a pressure higher than atmospheric pressure. For example, with respect to the temperature, any of the case of {[temperature of the reaction step (hereinafter referred to as "TA")] > [temperature of the intermediate processing step (hereinafter referred to as "TD")] > [temperature of the separation step (hereinafter referred to as "TB")]}, the case of (TA > TD < TB), and the case of (TA < TD < TB) may adapt to the invention. Also, in the relationship between the reaction step and the separation step, any of (TA < TB) and (TA > TB) may also adapt to the invention. With respect to the pressure, exactly the same is applicable, and any of the case of {[pressure in the reaction step (hereinafter referred to as "PA")] > [pressure in the intermediate processing step (hereinafter referred to as "PD")] > [pressure in the separation step (hereinafter referred to as "PB")]}, the case of (PA > PD < PB), the case of (PA < PD < PB), the case of (PA < PB), and the case of (PA > PB) may adapt to the invention. In addition, the relationship between the temperature and the pressure is not always required to work together. For example, by raising the pressure in the intermediate processing step and passing the resulting reaction product through a condenser, the temperature becomes (TA > TB), and the pressure becomes (PA < PB).

Next, the production process of the invention is applied to the case where the desired compound is recovered as a solid. With respect to the compound to be produced by the production process of the invention, ones that are stably present in the foregoing respective steps and in which at least a part thereof is present as a solid at least prior to the separation step may be employed. Aromatic carboxylic acids are preferable, aromatic dicarboxylic acids are more preferable, and terephthalic acid is especially preferable.

Also, the production process of the invention includes a step of solid-liquid separation. Since the desired material is recovered as a solid, it is preferable that an unreacted raw material itself is liquid or has high solubility in the reaction medium and/or the washing liquid because it can increase the purity of the desired compound .

As a preferred embodiment in the invention, there is enumerated liquid phase oxidation of an alkylbenzene with molecular oxygen. By this reaction, there are obtained aromatic carboxylic acids such as aromatic monocarboxylic acids, aromatic dicarboxylic acids, and aromatic tricarboxylic acids and those in which a part of the alkyl groups is oxidized. Production of terephthalic acid to which the process of the invention is applied will be hereunder described as a representative example. Incidentally, p-xylene is enumerated as the alkylbenzene as the raw material.

In the liquid phase oxidation of p-xylene, acetic acid is usually used as the reaction medium. An amount of the reaction medium to be used is usually from 1 to 10 times by weight, preferably from 2 to 8 times by weight, and more preferably from 3 to 6 times by weight based on the p-xylene. Also, the reaction medium may usually contain not more than 25 % by weight, and preferably not more than 10 % by weight of water in addition to acetic acid. Also, in the liquid phase oxidation reaction of p-xylene, water is formed. The moisture content in the reaction medium including water that is originally contained in the solvent is controlled such that it is usually from 5 to 25 % by weight, and preferably from 7 to 20 % by weight at the maximum. The adjustment of the moisture content can be carried out by purging a part of a condensed reflux liquid obtained by condensing a gas evaporated from the reactor outside the system according to the conventional manner.

Examples of the molecular oxygen-containing gas that is used for oxidation of p-xylene include air, oxygen-diluted air, and oxygen-rich air, with air being desirable from the viewpoints of equipment and costs.

Also, as the catalyst in the oxidation reaction, any known catalysts can be used. Typically, cobalt, manganese, and bromine are mainly used. Specific compounds of the catalyst component are as follows. Examples of cobalt compounds include cobalt acetate, cobalt naphthenate, and cobalt bromide, with cobalt acetate being preferable. Examples of manganese compounds include manganese acetate, manganese naphthenate, and manganese bromide, with manganese acetate being preferable. Also, examples of bromine compounds include hydrogen bromide, sodium bromide, cobalt bromide, manganese bromide, and bromoethane, with hydrogen bromide being preferable. These compounds may be used in combination. Also, other metal components may be present in the acetic acid solvent. For example, when the sodium component is present in an amount of from 1 ppm to 100 ppm, it further plays a role to prevent precipitation of the manganese component and has an effect to enhance the transmittance of the resulting terephthalic acid.

An amount of the catalyst to be used is as follows. The amount of the cobalt component to be used is from 100 ppm by weight to 2,000 ppm by weight, and preferably from 200 ppm by weight to 1,000 ppm by weight as reduced into cobalt metal based on the solvent. The amount of the manganese component to be used is from 1 ppm by weight to 1,000 ppm by weight, and preferably from 5 ppm by weight to 500 ppm by weight. The amount of the bromine compound to be used is from 400 ppm to 2,000 ppm.

Also, for the sake of promoting the reaction, a co-oxidizer can be used in combination.

Incidentally, the reaction medium contains reaction intermediates such as 4-carboxybenzaldehyde, p-toluic acid, p-tolualdehdye and impurities such as benzoic acid.

The oxidation reaction of p-xylene is carried out in the acetic acid solvent in the presence of a catalyst containing cobalt, manganese and bromine at a temperature of from 140 °C to 230 °C, and preferably from 150 °C to 210 °C while continuously feeding a molecular oxygen-containing gas, to oxidize 95 % or more of p-xylene. This is called as a first reaction zone. The reaction pressure is a pressure under which the mixture can keep the liquid phase at least at the reaction temperature or higher and is usually from 0.2 to 5 MPa. The reaction time of oxidation (mean residence time) is usually from 30 to 300 minutes.

In the first reaction zone, for the sake of increasing an oxygen partial pressure in the reaction gas phase portion, there may be employed a method in which the oxidation waste gas obtained by condensing and removing a condensing component from the gas discharged from the reactor is branched into two flows, and one of the flows is taken out from the system, whereas the other flow is continuously circulated and fed into the reactor.

Next, it is possible to provide a second reaction zone of from 130 °C to 240 °C, preferably from 140 °C to 220 °C, and more preferably from 160 °C to 200 °C as the need arises. In this reaction zone, low-temperature additional oxidation is carried out with molecular oxygen without adding p-xylene. The reaction time of the low-temperature additional oxidation (mean residence time) is usually from 20 to 90 minutes.

Next, it is also possible to carry out additional oxidation with molecular oxygen without adding p-xylene in a third reaction zone of from 150 to 300 °C, and preferably from 200 °C to 280 °C, the temperature of which is higher than that of the second reaction zone, as the need arises. The reaction time of the additional oxidation (mean residence time) is from 5 to 120 minutes.

The oxidation reaction may be terminated in the first reaction zone, or may be carried out until the second reaction zone or third reaction zone. According to the definition of the present description, the final reaction zone in the selected process is corresponding to the reactor 1 of Fig. 1. For example, if the oxidation reaction is carried out until the third reaction zone, the third reaction zone is corresponding to the reactor 1 of Fig. 1.

The slurry to be introduced into the intermediate processing step through the line 12 of Fig. 1 becomes from 130 °C to 300 °C according to the foregoing illustration. This intermediate processing may be carried out, or the slurry may be transferred directly into the separation step 3 through the line 13 without carrying out the intermediate processing. For example, in the example of terephthalic acid, in the case where the reaction is carried out until the second reaction zone, the slurry of from 130 °C to 240 °C is obtained. However, since crystallization is not required, the resulting slurry is transferred into the separation device while keeping the reaction temperature. The pressure of the slurry is equal to or higher than the vapor pressure of the mother liquor in the slurry. Since the temperature decreases with a decrease of the pressure, the temperature is actually controlled by operating the pressure of the slurry.

With respect to the slurry to be fed into the separation device through the line 13 of Fig. 1, the temperature is determined while taking into consideration the amount of the cake-attached liquid to be evaporated from the cake. For example, when a mixed solution having a water concentration of 10 % by weight and an acetic acid concentration of 90 % by weight in the reaction medium is attached in a weight ratio of 11 % to the cake of terephthalic acid, the cake temperature necessary for completely evaporating the cake-attached mother liquor is 170 °C or higher. Accordingly, though the slurry obtained by carrying out the reaction until the second reaction zone is, for example, from 130 °C to 240 °C, it is preferable to carry out the reaction in the second reaction zone at a temperature higher than 170 °C. Incidentally, even if the reaction is carried out at a temperature lower than 170 °C, by keeping a temperature at higher than 110 °C as the boiling point at atmospheric pressure, it is possible to industrially utilize a difference from the actual cake temperature as a sensible heat for drying the cake.

Accordingly, it has an important meaning from the viewpoint of energy utilization in the invention to keep the temperature at the boiling point at normal pressure or higher without releasing the slurry pressure in the process after the reaction to the atmospheric pressure. However, in the illustrated case, in the case where only a slurry of not higher than 170 °C is obtained, the quantity of heat insufficient for drying the cake must be replenished, and heating is carried out for any one of the slurry before separation, the cake after separation, or the cake after discharge. In the slurry before separation, use of a heat exchanger may be enumerated; in the cake after separation, heating of the washing liquid or heating of the gas atmosphere may be enumerated; and in the cake after discharge, heating from the outside of the powder tank or heat renewal of the gas atmosphere may be enumerated. Incidentally, for the sake of keeping the slurry at 170 °C or higher, a pressure of 0.6 MPa or more is required. The pressure of the separator is preferably from 0.65 MPa to 1.5 MPa, more preferably from 0.7 MPa to 1.3 MPa, and especially preferably from 0.8 MPa to 1.0 MPa.

Incidentally, in order that the cake washing liquid does not carry away the heat energy that the cake has, it is preferable that the cake washing liquid has a temperature equal to or higher than the temperature of the cake. As a specific example, when the cake temperature is 170 °C, a washing liquid of 170 °C or higher containing acetic acid as the major component, such as one of 5 °C or higher than 170 °C, is prepared. Additionally, for the sake of preventing bumping of the washing liquid, the pressure within the separation device into which the washing liquid is introduced is a vapor pressure of the washing liquid or higher. For example, it is preferable that the pressure within the separation device is higher than the vapor pressure of the slurry to be fed into the separation device by 0.01 to 1.0 MPa and that the vapor pressure of the washing liquid falls within this range. With respect to the kind of the cake washing liquid, it is preferable that the total sum of evaporation latent heats of the respective components is not higher than 300 kcal/kg. Though water as a single body has an evaporation latent heat of 500 kcal/kg or higher, if it has an average value of evaporation latent heat of 300 kcal/kg as a mixture, even ones containing water are preferably used. Also, the composition of the washing liquid may be quite different from the reaction medium. For example, in the case of the reaction medium containing acetic acid as the major component as illustrated above, the washing liquid may be, for example, a quite different, pure substance of an acetic acid ester, or may have exactly the same component as the reaction medium except for impurities.

The terephthalic acid cake having come out from the separation device is resident in the chamber 4 illustrated in Fig. 1. The cake is intermittently or continuously discharged into the powder tank at atmospheric pressure. An average value of the residence amount of the cake in the chamber 4 is preferably, for example, from 0.0001 to 0.1 in terms of a weight ratio when the processing amount is defined as 1 per hour, and relies upon the chamber shape, the upper limit of the cake amount, and the lower limit of the case amount. The discharge amount is determined from the upper limit and lower limit and the residence amount of the cake in the chamber 4. For example, when the processing amount is defined as 1 per hour, the discharge amount is from 0.002 to 0.02 per one-time drive of the valve 5. Incidentally, the drive distance of the valve 5 is preferably from 3 to 50 mm, and more preferably from 10 to 25 mm. Also, the drive frequency is preferably from 50 to 500 times per hour. When the drive distance and the drive frequency fall within the preferred ranges, the discharge of the compound into the compound recovery zone from the separation device becomes good.

If desired, the terephthalic acid cake discharged into the powder tank 6 illustrated in Fig. 1 is, for example, subjected to removal of a trace amount of the cake-attached liquid with a dry gas, or the like and then recovered from the line 15. Also, the gas containing acetic acid as the major component as generated within the atmospheric system and the dry gas are removed through the line 14. The cake recovered through the line 15 further passes through a purification step to become a product as the need arises.

### <Examples>

The invention will be more specifically described below with reference to the Examples. However, it should be construed that the invention is never limited to these Examples.

### Comparative Example 1:

In equipment with an output of terephthalic acid of 36 ton/hr, p-xylene, acetic acid in an amount of 5.5 times by weight based on the p-xylene, cobalt acetate, manganese acetate, and hydrogen bromide were continuously fed into a liquid phase oxidation reaction vessel and subjected to oxidation reaction at a temperature of 197 °C under a pressure of 1.45 MPa for a reaction time (mean residence time) of 90 minutes. The amount of the catalysts to be used is 280 ppm by weight for the cobalt component as reduced to cobalt metal, 280 ppm by weight for the manganese component, and 700 ppm by weight for the bromine component, based on the solvent, respectively.

Air is used as a gas for undergoing the oxidation reaction with molecular oxygen. At this time, the oxygen content of air is 21 %. And compressed air was fed into the reactor such that the oxygen concentration in the gas discharged from the reactor (this gas being hereinafter sometimes referred to as "waste gas") was 5 % by volume. Next, the oxidized slurry was continuously transferred into a low-temperature additional oxidation reaction vessel, and air (oxygen content: 21 %) was fed as a gas for carrying out oxidation reaction at a temperature of 190 °C under a pressure of 1.3 MPa for a reaction time (mean residence time) of 35 minutes such that the oxygen concentration in the waste gas was 6 % by volume, to undergo low-temperature additional oxidation.

The slurry additionally oxidized at low temperatures was continuously crystallized in a three-stage intermediate processing tank and subjected to solid-liquid separation at atmospheric pressure. At this time, the liquid content of the cake having been subjected to solid-liquid separation was 15.0 %.

Next, this cake was dried at atmospheric pressure in a steam tube dryer type drying machine using steam as a heating source. Steam having a pressure of 0.4 MPa is circulated around the drying machine, whereby a heating furnace is heated. The cake-attached liquid evaporated upon heating is taken out from the system with a nitrogen gas circulated within the drying machine. Also, an outlet temperature of the drying machine is 140 °C.

When one ton of terephthalic acid was dried using this drying machine, the quantity of heat of steam used in the drying machine was 36,000 kcal. Also, the liquid content of the resulting terephthalic acid was 0.1 %.

Accordingly, in the case where the terephthalic acid cake is dried at atmospheric pressure from the liquid content of 15.0 % to the liquid content of 0.1 %, the quantity of heat of 36,000 kcal per one ton of terephthalic acid is necessary.

### Example 1:

Next, in the production process of the invention, that is, in the case where a solid and a liquid are separated while keeping the pressure and temperature generated in the reaction step, and the resulting cake is transferred into a zone lower than the separation zone, an experiment was performed in order to verify an effect of evaporating and separating the cake-attached liquid utilizing a difference in a sensible heat as generated.

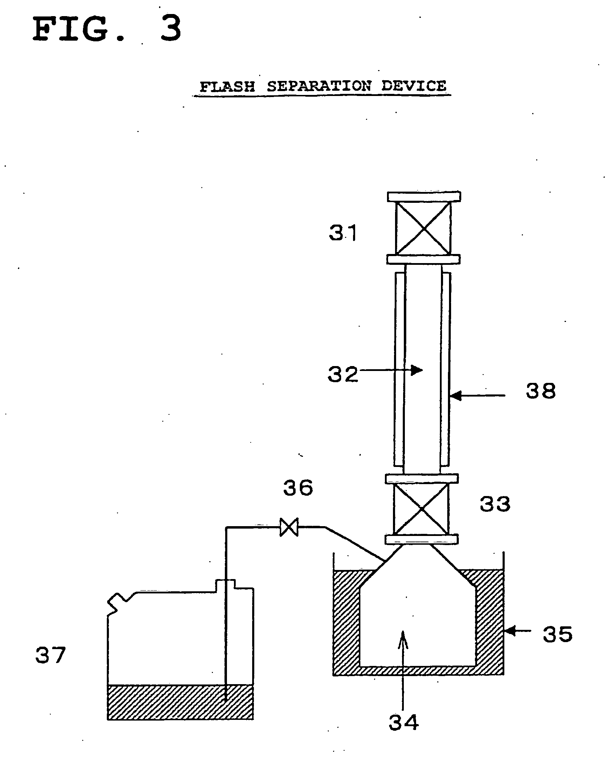

A cake obtained in the same manner as in Comparative Example was dried by flash separation using the equipment shown in Fig. 3.

The flash separation device is provided with a heater 32 having an internal volume of 1.2 liters (a cylinder having an inner diameter of 40 mm and a length of 800 mm), a receiver 34 (compound recovery zone) having an internal volume of 3 liters, and a jacket 38 for heating the heater, and the receiver can be dipped in an oil bath 35 for heating or cooling.

A cake having a liquid content 14.1 % (corresponding to 700 g in terms of the solids content; composition of the cake-attached liquid having a water concentration of 10 % and an acetic acid concentration of 90 %) as obtained in the same manner as in Comparative Example 1 was charged at atmospheric pressure into the heater 32 through an upper valve 31. If the cake is heated as it is, a part of acetic acid and water attached to the cake are evaporated so that the liquid content of the cake changes. Accordingly, a corresponding amount was previously added to the heater, and the heater was then sealed and raised to 173 °C. At this time, the pressure of the heater was 0.6 MPa.

In the receiver positioned below the heater, for the sake of rendering the inside thereof into an acetic acid atmosphere, the inside was evaporated, and acetic acid in an amount corresponding to that volume was previously charged. The receiver was then dipped in a heat medium oil of 115 °C. And a bottom valve 33 was opened, and the cake and the vapor evaporated from the attached liquid within the heater were transferred into the receiver at atmospheric pressure.

Since the pressure of the heater instantly became atmospheric pressure, not only the bottom valve of the heater was immediately closed, but also a valve 36 between the receiver and a trap vessel 37 charged with ethylene glycol was closed. Since the temperature within the receiver became stable at 123 °C, cooling was performed.

After flash separation, if the receiver is opened to the air, since diffusion of the liquid component remaining on the case surface into the air is fast, it is difficult to measure a true value. For this reason, the receiver was cooled in a closed system, the gas component in the vapor phase portion of the receiver was condensed, specifically sufficiently cooled to 20 °C at which vapors of acetic acid and water are not substantially generated, and the whole of cake containing acetic acid present within the receiver was then recovered.

It was confirmed that the results obtained in a method of determining the amount of the cake-attached liquid from the weight loss after drying the cake are consistent with those obtained by determining the amount of the cake-attached liquid by analyzing the composition in the liquid used for washing the cake surface with water.

The vapors of acetic acid and water to be discharged through the receiver by flash separation were introduced into a vessel charged with ethylene glycol or water and absorbed therein, and an increase of the weights of acetic acid and water and the liquid component in the receiver were then analyzed by gas chromatography to confirm a mass balance.

A value obtained by subtracting the amount of acetic acid present in the vapor phase portion of the receiver before cooling the receiver from the amount of the cake-attached liquid thus determined was taken as an amount of the liquid attached to the cake immediately after the flash separation, to determine a liquid content. Incidentally, this cake, had a median diameter of 99 µm. A laser scattering type particle size distribution analyzer, "Lazer Micron Sizer/LMS-24" manufactured by Seishin Enterprise Co., Ltd. was used for analysis of the median diameter. One gram of the recovered cake and a surfactant were charged in a water-filled sample chamber of this device. Thereafter, dispersion under ultrasonic wave for 30 seconds and analysis by laser scattering automatically proceed. After completion of the analysis, a volume frequency distribution is obtained every respective particle size interval. A particle size in which the cumulative frequency is 50 % was calculated from the results.

### Example 2:

Flash separation was carried out in the same manner as in Example 1, except that in Example 1, the cake-attached liquid had a composition having a water concentration of 10 % and an acetic acid concentration of 90 %, the liquid content of the cake was 10.4 %, and the temperature of the heater was 187 °C. After flash separation, since the temperature within the receiver became stable at 110 °C, cooling was performed.

### Example 3:

Flash separation was carried out in the same manner as in Example 1, except that in Example 1, the cake-attached liquid had a composition having an acetic acid concentration of 100 %, the liquid content of the cake was 15.0 %, and the temperature of the heater was 144 °C.

In Examples 1 to 3, the operation conditions are shown in Table 1, and the results are shown together in Table 2.

In any of the cases of Examples 1 to 3, it is assumed that the separation step and the drying step are carried out while keeping the temperature and the pressure generated in the reaction step. Accordingly, no new energy is used in the drying step other than the temperature and the pressure generated in the reaction step. The drying is respectively achieved without adding a new quantity of heat such that in Example 1, the liquid content changes from 14.1 % to 1.3 %, in Example 2, the liquid content changes from 10.4 % to 0.1 %, and in Example 3, the liquid content changes from 15.0 % to 5.7 %.

Also, as shown in Table 2, the liquid contents of cake after the flash separation were consistent with those calculated from enthalpies that the systems have. Accordingly, it was revealed that by employing the production process of the invention, the internal energy to be brought by the reaction step can be effectively utilized in the drying step, thereby enabling to reduce a large quantity of heat energy necessary in the conventional drying step.

### Table 1

**Table 1**

| Example | Cake before flash separation | | Pressure within heater before flash operation (MPa) | Pressure within receiver immediately after flash operation (MPa) | Difference in temperature in flash separation operation Heater → Receiver (°C) |
|---|---|---|---|---|---|
| | Composition of cake-attached liquid (%) | Liquid content (%) | | | |
| 1 | Acetic acid 90 - Water 10 | 14.1 | 0.60 | 0.1 | 173 → 123 |
| 2 | Acetic acid 90 - Water 10 | 10.4 | 0.85 | 0.1 | 187 → 112 |
| 3 | Acetic acid | 15.0 | 0.20 | 0.1 | 144 → 118 |

### Table 2

**Table 2**

| Example | Calculated value of liquid content of cake after flash separation (%) | Found value of liquid content of cake after flash separation (%) |
|---|---|---|
| 1 | 1.1 | 1.3 |
| 2 | 0 | 0.1 |
| 3 | 5.2 | 5.7 |

A large quantity of heat is necessary in a method of re-heating and drying after solid-liquid separation at atmospheric pressure. This is because it is required to heat terephthalic acid itself in addition to the mother liquor medium to be evaporated. As compared with this method, the method of drying under pressure after solid-liquid separation while keeping the temperature at atmospheric pressure or higher enables to greatly reduce energy to be consumed in drying.

## Claims

1. A process of producing a compound, including:
(A) a reaction step, wherein a raw material and a reaction medium are fed into a reactor (1) and wherein in the reactor (1) a reaction is carried out under a pressure higher than atmospheric pressure and at a temperature of the boiling point at atmospheric pressure of the reaction medium or higher, to form a compound,
wherein the reaction medium is one that becomes a liquid phase system or a gas-liquid two-phase system during the reaction step, and one that does not chemically change the reaction substrate or the desired compound after the reaction;
(B) a separation step, wherein in a separation device (3) a slurry containing the compound and the reaction medium is subjected to solid-liquid separation under a pressure higher than atmospheric pressure and at a temperature of the boiling point at atmospheric pressure of the reaction medium or higher, to obtain a cake having a weight ratio of a cake-attached liquid of not more than 50 %, based on the solids content, wherein the cake-attached liquid is the sum of the reaction medium and a washing liquid in the optional case of washing the cake;
(C) a drying step, wherein the resulting cake is moved into a compound recovery zone (6) having a pressure lower than the pressure in the separation device (3) and a temperature lower than the temperature in the separation device (3), thereby evaporating the cake-attached liquid by internal energy released during the movement, wherein in the drying step (C), the weight ratio of the cake-attached liquid is not more than 10 % based on the solids content; and
(D) optionally an intermediate processing step between the reaction step (A) and the separation step (B);
wherein the steps from the reactor (1) until the separation device (3) optionally through an intermediate processing tank (2) meet the requirements that the pressure be kept higher than atmospheric pressure, and the temperature be kept at the boiling point at atmospheric pressure of the reaction medium or higher.

2. The process of producing a compound according to claim 1, wherein in the separation step (B), the cake is washed with a washing liquid having an evaporation latent heat at the boiling point at atmospheric pressure of not more than 300 kcal/kgin a state in which the pressure is kept at higher than atmospheric pressure, and the temperature is kept at the boiling point at atmospheric pressure of the reaction medium or higher.

3. The process of producing a compound according to claim 1 or 2, wherein the reaction medium has an evaporation latent heat at the boiling point at atmospheric pressure of not more than 300 kcal/kg.

4. The process of producing a compound according to any one of claims 1 to 3, wherein in the separation step (B), the cake is washed with a washing liquid having a temperature in the range of the boiling point at atmospheric pressure of the washing liquid or higher but not higher than (TB1 + 100°C) (wherein TB1 stands for the temperature (°C) of an unwashed cake).

5. The process of producing a compound according to any one of claims 1 to 4, wherein in the separation step (B), the cake is washed with a washing liquid in an amount of from 0.03 to 5.0 times based on the weight of the solids content in the cake.

6. The process of producing a compound according to any one of claims 1 to 5, wherein the compound to be formed in the reaction step (A) is an aromatic carboxylic acid.

7. The process of producing a compound according to claim 6, wherein the aromatic carboxylic acid is terephthalic acid.

8. The process of producing a compound according to claim 6, wherein in the reaction step (A), an alkyl group-substituted aromatic compound is subjected to liquid phase oxidation with molecular oxygen to obtain the aromatic carboxylic acid.

9. The process of producing a compound according to claim 7, wherein in the reaction step (A), p-xylene is subjected to liquid phase oxidation with molecular oxygen to obtain terephthalic acid.

10. The process of producing a compound according to any one of claims 1 to 9, wherein the reaction step (A) is carried out at a temperature in the range of from 50 °C to 350 °C.

11. The process of producing a compound according to any one of claims 1 to 10, wherein the reaction step (A) is carried out under a pressure in the range of exceeding atmospheric pressure but not higher than 20 MPa.

12. The process of producing a compound according to any one of claims 1 to 11, wherein in the drying step (C), a difference between the temperature of the cake within the separation device (3) and the temperature of the cake discharged into the compound recovery zone (6) is from 5°C to 250°C.

13. The process of producing a compound according to any one of claims 1 to 12, wherein in the drying step (C), a difference between the pressure within the separation device (3) and the pressure in the compound recovery zone (6) is from 0.01 MPa to 22 MPa.

14. The process of producing a compound according to any one of claims 1 to 13, wherein in the drying step (C), the compound to be discharged has a median diameter of from 40 µm to 300 µm.

15. The process of producing a compound according to any one of claims 1 to 14, wherein in the drying step (C), the weight ratio of the cake-attached liquid is reduced by 3 % or more based on the solids content.

16. The process of producing a compound according to any one of claims 1 to 15, wherein in the drying step (C), an intermediate chamber (16) is provided between the separation device (3) and the compound recovery zone (6).

17. The process of producing a compound according to any one of claims 1 to 16, wherein in the drying step (C), a dry gas is introduced into the intermediate chamber (16) and/or the compound recovery zone (6).

18. The process of producing a compound according to any one of claims 1 to 17, wherein in the drying step (C), the pressure in the compound recovery zone (6) is atmospheric pressure.

19. The process of producing a compound according to any one of claims 1 to 18, wherein in the drying step (C), a pressure drying device provided with a discharge valve is used.

20. The process of producing a compound according to claim 19, wherein a contact portion between a valve body and a valve seat of the discharge valve is linear, and its shape is circular.

21. The process of producing a compound according to claim 19 or 20, wherein in the drying step (C), the discharge valve is intermittently opened, and an opening time is from 0.01 seconds to 1 second.

22. The process of producing a compound according to any one of claims 1 to 21, wherein an intermediate processing step (D) for carrying out crystallization or dissolution of the compound is provided between the reaction step (A) and the separation step (B).

23. The process of producing a compound according to any one of claims 1 to 22, wherein in the reaction step (A), the formed compound is obtained as a solid.

## Patentansprüche

1. Ein Verfahren zur Herstellung einer Verbindung, umfassend:
(A) einen Reaktionsschritt, bei dem ein Ausgangsmaterial und ein Reaktionsmedium einem Reaktor (1) zugeführt werden und bei dem in dem Reaktor (1) eine Reaktion unter einem Druck, der höher ist als Atmosphärendruck, und bei einer Temperatur des Siedepunkts bei Atmosphärendruck des Reaktionsmediums oder höher durchgeführt wird, um eine Verbindung zu bilden,
wobei das Reaktionsmedium eines ist, das während des Reaktionsschrittes ein Flüssigphasensystem oder ein Gas-Flüssig-Zweiphasensystem wird, und eines ist, das das Reaktionssubstrat oder die gewünschte Verbindung nach der Reaktion nicht chemisch verändert;
(B) einen Trennschritt, bei dem in einer Trennvorrichtung (3) eine die Verbindung und das Reaktionsmedium enthaltende Aufschlämmung einer Feststoff-Flüssigkeits-Trennung unter einem Druck höher als Atmosphärendruck und bei einer Temperatur des Siedepunkts bei Atmosphärendruck des Reaktionsmediums oder höher unterzogen wird, um einen Kuchen mit einem Gewichtsverhältnis einer dem Kuchen anhaftenden Flüssigkeit von nicht mehr als 50%, bezogen auf den Feststoffgehalt, zu erhalten, wobei die dem Kuchen anhaftende Flüssigkeit die Summe des Reaktionsmediums und einer Waschflüssigkeit im optionalen Fall des Waschens des Kuchens ist;
(C) einen Trocknungsschritt, bei dem der resultierende Kuchen in eine Zone (6) zur Gewinnung einer Verbindung, welche einen niedrigeren Druck als der Druck in der Trennvorrichtung (3) und eine niedrigere Temperatur als die Temperatur in der Trennvorrichtung (3) aufweist, überführt wird, wodurch die dem Kuchen anhaftende Flüssigkeit durch die während der Überführung freigesetzte innere Energie verdampft,
wobei in dem Trocknungsschritt (C) das Gewichtsverhältnis der dem Kuchen anhaftenden Flüssigkeit nicht mehr als 10%, bezogen auf den Feststoffgehalt, beträgt; und
(D) gegebenenfalls einen dazwischenliegenden Verarbeitungsschritt zwischen dem Reaktionsschritt (A) und dem Trennschritt (B);
wobei die Schritte vom Reaktor (1) bis zur Trennvorrichtung (3) gegebenenfalls durch einen dazwischenliegenden Verarbeitungsbehälter (2) die Anforderungen erfüllen, dass der Druck höher als Atmosphärendruck gehalten wird und die Temperatur beim Siedepunkt bei Atmosphärendruck des Reaktionsmediums oder höher gehalten wird.

2. Das Verfahren zur Herstellung einer Verbindung nach Anspruch 1, wobei in dem Trennschritt (B) der Kuchen mit einer Waschflüssigkeit mit einer latenten Verdampfungswärme beim Siedepunkt bei Atmosphärendruck von nicht mehr als 300 kcal/kg in einem Zustand, in dem der Druck höher als Atmosphärendruck gehalten wird und die Temperatur beim Siedepunkt bei Atmosphärendruck des Reaktionsmediums oder höher gehalten wird, gewaschen wird.

3. Das Verfahren zur Herstellung einer Verbindung nach Anspruch 1 oder 2, wobei das Reaktionsmedium eine latente Verdampfungswärme beim Siedepunkt bei Atmosphärendruck von nicht mehr als 300 kcal/kg aufweist.

4. Das Verfahren zur Herstellung einer Verbindung nach einem der Ansprüche 1 bis 3, wobei in dem Trennschritt (B) der Kuchen mit einer Waschflüssigkeit gewaschen wird, welche eine Temperatur im Bereich des Siedepunkts bei Atmosphärendruck der Waschflüssigkeit oder höher, jedoch nicht höher als (TB 1 + 100°C) (wobei TB 1 für die Temperatur (°C) eines ungewaschenen Kuchens steht) aufweist.

5. Das Verfahren zur Herstellung einer Verbindung nach einem der Ansprüche 1 bis 4, wobei in dem Trennschritt (B) der Kuchen mit einer Waschflüssigkeit in einer Menge von dem 0,03- bis 5,0-Fachen, bezogen auf das Gewicht des Feststoffgehalts in dem Kuchen, gewaschen wird.

6. Das Verfahren zur Herstellung einer Verbindung nach einem der Ansprüche 1 bis 5, wobei die in dem Reaktionsschritt (A) zu bildende Verbindung eine aromatische Carbonsäure ist.

7. Das Verfahren zur Herstellung einer Verbindung nach Anspruch 6, wobei die aromatische Carbonsäure Terephthalsäure ist.

8. Das Verfahren zur Herstellung einer Verbindung nach Anspruch 6, wobei in dem Reaktionsschritt (A) eine mit Alkylresten substituierte aromatische Verbindung einer Flüssigphasenoxidation mit molekularem Sauerstoff unterzogen wird, um die aromatische Carbonsäure zu erhalten.

9. Das Verfahren zur Herstellung einer Verbindung nach Anspruch 7, wobei in dem Reaktionsschritt (A) p-Xylol einer Flüssigphasenoxidation mit molekularem Sauerstoff unterzogen wird, um Terephthalsäure zu erhalten.

10. Das Verfahren zur Herstellung einer Verbindung nach einem der Ansprüche 1 bis 9, wobei der Reaktionsschritt (A) bei einer Temperatur im Bereich von 50°C bis 350°C durchgeführt wird.

11. Das Verfahren zur Herstellung einer Verbindung nach einem der Ansprüche 1 bis 10, wobei der Reaktionsschritt (A) unter einem Druck im Bereich von Über-Atmosphärendruck, jedoch nicht höher als 20 MPa, durchgeführt wird.

12. Das Verfahren zur Herstellung einer Verbindung nach einem der Ansprüche 1 bis 11, wobei in dem Trocknungsschritt (C) ein Unterschied zwischen der Temperatur des Kuchens in der Trennvorrichtung (3) und der Temperatur des in die Zone (6) zur Gewinnung einer Verbindung überführten Kuchens 5°C bis 250°C beträgt.

13. Das Verfahren zur Herstellung einer Verbindung nach einem der Ansprüche 1 bis 12, wobei in dem Trocknungsschritt (C) ein Unterschied zwischen dem Druck in der Trennvorrichtung (3) und dem Druck in der Zone (6) zur Gewinnung einer Verbindung 0,01 MPa bis 22 MPa beträgt.

14. Das Verfahren zur Herstellung einer Verbindung nach einem der Ansprüche 1 bis 13, wobei in dem Trocknungsschritt (C) die zu überführende Verbindung einen mittleren Durchmesser von 40 µm bis 300 µm aufweist.

15. Das Verfahren zur Herstellung einer Verbindung nach einem der Ansprüche 1 bis 14, wobei in dem Trocknungsschritt (C) das Gewichtsverhältnis der dem Kuchen anhaftenden Flüssigkeit um 3% oder mehr, bezogen auf den Feststoffgehalt, verringert wird.

16. Das Verfahren zur Herstellung einer Verbindung nach einem der Ansprüche 1 bis 15, wobei in dem Trocknungsschritt (C) eine dazwischenliegende Kammer (16) zwischen der Trennvorrichtung (3) und der Zone (6) zur Gewinnung einer Verbindung bereitgestellt ist.

17. Das Verfahren zur Herstellung einer Verbindung nach einem der Ansprüche 1 bis 16, wobei in dem Trocknungsschritt (C) ein trockenes Gas in die dazwischenliegende Kammer (16) und/oder die Zone (6) zur Gewinnung einer Verbindung eingeleitet wird.

18. Das Verfahren zur Herstellung einer Verbindung nach einem der Ansprüche 1 bis 17, wobei in dem Trocknungsschritt (C) der Druck in der Zone (6) zur Gewinnung einer Verbindung Atmosphärendruck ist.

19. Das Verfahren zur Herstellung einer Verbindung nach einem der Ansprüche 1 bis 18, wobei in dem Trocknungsschritt (C) eine mit einem Auslassventil versehene Drucktrocknungsvorrichtung verwendet wird.

20. Das Verfahren zur Herstellung einer Verbindung nach Anspruch 19, wobei ein Kontaktbereich zwischen einem Ventilkörper und einem Ventilsitz des Auslassventils linear ist und seine Form kreisförmig ist.

21. Das Verfahren zur Herstellung einer Verbindung nach Anspruch 19 oder 20, wobei in dem Trocknungsschritt (C) das Auslassventil in Abständen geöffnet wird und eine Öffnungszeit 0,01 Sekunden bis 1 Sekunde beträgt.

22. Das Verfahren zur Herstellung einer Verbindung nach einem der Ansprüche 1 bis 21, wobei ein dazwischenliegender Verarbeitungsschritt (D) zur Durchführung einer Kristallisation oder Lösung der Verbindung zwischen dem Reaktionsschritt (A) und dem Trennschritt (B) bereitgestellt ist.

23. Das Verfahren zur Herstellung einer Verbindung nach einem der Ansprüche 1 bis 22,
wobei in dem Reaktionsschritt (A) die gebildete Verbindung als Feststoff erhalten wird.

## Revendications

1. Procédé de production d'un composé, comprenant :
(A) une étape de réaction au cours de laquelle un matériau brut et un milieu de réaction sont introduits dans un réacteur (1) et au cours de laquelle on conduit dans le réacteur (1) une réaction sous une pression supérieure à la pression atmosphérique et à la température du point d'ébullition à pression atmosphérique du milieu de réaction ou à une température plus élevée, pour former un composé,
le milieu de réaction étant un milieu de réaction qui devient un système en phase liquide ou un système en deux phases gaz-liquide pendant l'étape de réaction, le milieu de réaction ne modifiant pas chimiquement le substrat de réaction ou le composé que l'on souhaite obtenir après la réaction,
(B) une étape de séparation au cours de laquelle dans un dispositif de séparation (3) une suspension qui contient le composé et le milieu de réaction est soumise à une séparation solide-liquide sous une pression supérieure à la pression atmosphérique et à la température du point d'ébullition à pression atmosphérique du milieu de réaction ou à une température plus élevée, pour obtenir un gâteau dont le rapport pondéral d'un liquide lié au gâteau relatif aux solides n'est pas supérieur à 50 %, le liquide lié au gâteau étant la somme du milieu de réaction et d'un liquide de nettoyage si l'on effectue facultativement un nettoyage du gâteau,
(C) une étape de séchage au cours de laquelle le gâteau obtenu est déplacé dans une zone (6) de récupération d'un composé dont la pression est inférieure à la pression du dispositif de séparation (3) et la température est inférieure à la température du dispositif de séparation (3), ce qui permet d'évaporer le liquide lié au gâteau par l'énergie interne libérée pendant le déplacement,
le rapport pondéral du liquide lié au gâteau relatif aux solides n'étant pas supérieur à 10 % lors de l'étape de séchage (C), et
(D) une étape intermédiaire de traitement réalisée facultativement entre l'étape de réaction (A) et l'étape de séparation (B),
les étapes se déroulant entre le réacteur (1) et le dispositif de séparation (3) pouvant facultativement se dérouler au travers d'un réservoir (2) de traitement intermédiaire et remplissant les conditions que la pression soit maintenue à une valeur supérieure à la pression atmosphérique et la température soit maintenue au point d'ébullition à pression atmosphérique du milieu de réaction ou à une température plus élevée.

2. Procédé de production d'un composé selon la revendication 1, dans lequel lors de l'étape de séparation (B), le gâteau est nettoyé avec un liquide de nettoyage dont la chaleur latente d'évaporation au point d'ébullition à pression atmosphérique n'est pas supérieure à 300 kcal/kg, dans des conditions telles que la pression est maintenue à une pression supérieure à la pression atmosphérique et la température est maintenue au point d'ébullition à pression atmosphérique du milieu de réaction ou à une température plus élevée.

3. Procédé de production d'un composé selon la revendication 1 ou 2, dans lequel la chaleur latente d'évaporation du milieu de réaction au point d'ébullition à pression atmosphérique n'est pas supérieure à 300 kcal/kg.

4. Procédé de production d'un composé selon l'une quelconque des revendications 1 à 3, dans lequel lors de l'étape de séparation (B), le gâteau est nettoyé avec un liquide de nettoyage dont la température est comprise dans une plage qui va du point d'ébullition à pression atmosphérique du liquide de nettoyage à une température plus élevée mais qui n'est pas supérieure à (TB1 + 100°C) (où TB1 représente la température (°C) d'un gâteau non nettoyé).

5. Procédé de production d'un composé selon l'une quelconque des revendications 1 à 4, dans lequel au cours de l'étape de séparation (B), le gâteau est nettoyé avec une quantité de liquide de nettoyage se situant dans la plage de 0,03 à 5,0 fois la teneur pondérale en solides du gâteau.

6. Procédé de production d'un composé selon l'une quelconque des revendications 1 à 5, dans lequel le composé à former lors de l'étape de réaction (A) est un acide carboxylique aromatique.

7. Procédé de production d'un composé selon la revendication 6, dans lequel l'acide carboxylique aromatique est l'acide téréphtalique.

8. Procédé de production d'un composé selon la revendication 6, dans lequel lors de l'étape de réaction (A), un composé aromatique substitué par un groupe alkyle subit une oxydation en phase liquide avec de l'oxygène moléculaire afin d'obtenir l'acide carboxylique aromatique.

9. Procédé de production d'un composé selon la revendication 7, dans lequel lors de l'étape de réaction (A), du p-xylène subit une oxydation en phase liquide avec de l'oxygène moléculaire afin d'obtenir de l'acide téréphtalique.

10. Procédé de production d'un composé selon l'une quelconque des revendications 1 à 9, dans lequel l'étape de réaction (A) est réalisée à une température comprise dans la plage de 50°C à 350°C.

11. Procédé de production d'un composé selon l'une quelconque des revendications 1 à 10, dans lequel l'étape de réaction (A) est réalisée à une pression comprise dans la plage qui va d'au dessus de la pression atmosphérique à au plus 20 MPa.

12. Procédé de production d'un composé selon l'une quelconque des revendications 1 à 11, dans lequel lors de l'étape de séchage (C), une différence entre la température du gâteau présent dans le dispositif de séparation (3) et la température du gâteau déversé dans la zone (6) de récupération d'un composé est de 5°C à 250°C.

13. Procédé de production d'un composé selon l'une quelconque des revendications 1 à 12, dans lequel lors de l'étape de séchage (C), une différence entre la pression dans le dispositif de séparation (3) et la pression dans la zone (6) de récupération d'un composé est de 0,01 MPa à 22 MPa.

14. Procédé de production d'un composé selon l'une quelconque des revendications 1 à 13, dans lequel le composé déversé lors de l'étape de séchage (C) présente un diamètre moyen de 40 µm à 300 µm.

15. Procédé de production d'un composé selon l'une quelconque des revendications 1 à 14, dans lequel lors de l'étape de séchage (C), le rapport pondéral du liquide lié au gâteau relatif aux solides est abaissé de 3 % ou plus.

16. Procédé de production d'un composé selon l'une quelconque des revendications 1 à 15, dans lequel lors de l'étape de séchage (C), une chambre intermédiaire (16) est prévue entre le dispositif de séparation (3) et la zone (6) de récupération d'un composé.

17. Procédé de production d'un composé selon l'une quelconque des revendications 1 à 16, dans lequel lors de l'étape de séchage (C), un gaz sec est introduit dans la chambre intermédiaire (16) et/ou dans la zone (6) de récupération d'un composé.

18. Procédé de production d'un composé selon l'une quelconque des revendications 1 à 17, dans lequel lors de l'étape de séchage (C), la pression de la zone (6) de récupération d'un composé est la pression atmosphérique.

19. Procédé de production d'un composé selon l'une quelconque des revendications 1 à 18, dans lequel lors de l'étape de séchage (C), un dispositif de séchage sous pression doté d'un clapet de décharge est utilisé.

20. Procédé de production d'un composé selon la revendication 19, dans lequel une zone de contact entre un corps de clapet et un siège du clapet de décharge est linéaire et présente une forme circulaire. ,

21. Procédé de production d'un composé selon la revendication 19 ou 20, dans lequel lors de l'étape de séchage (C), le clapet de décharge est ouvert de façon intermittente, la durée d'ouverture étant de 0,01 seconde à 1 seconde.

22. Procédé de production d'un composé selon l'une quelconque des revendications 1 à 21, dans lequel une étape (D) de traitement intermédiaire qui permet de réaliser une cristallisation ou une dissolution du composé est prévue entre l'étape de réaction (A) et l'étape de séparation (B).

23. Procédé de production d'un composé selon l'une quelconque des revendications 1 à 22, dans lequel lors de l'étape de réaction (A), le composé formé est obtenu sous forme solide.
